# EUROPEAN PATENT SPECIFICATION

(11) **EP 3 229 881 B1**
(45) Date of publication and mention of the grant of the patent: **20.01.2021**
(21) Application number: 15823557.2
(22) Date of filing: 08.12.2015
(51) Int. Cl.: A61M 16/06

(54) **FACIAL MASK WITH INTERNAL SUPPORT STRUCTURE FOR USE WITH VENTILATION AND POSITIVE AIR PRESSURE SYSTEMS**
GESICHTSMASKE MIT INTERNER STÜTZSTRUKTUR ZUR VERWENDUNG MIT BELÜFTUNG UND POSITIVLUFTDRUCKSYSTEMEN
MASQUE FACIAL AYANT UNE STRUCTURE DE SUPPORT INTERNE DESTINÉ À ÊTRE UTILISÉ AVEC DES SYSTÈMES DE VENTILATION ET DE PRESSION D'AIR POSITIVE

(30) Priority: 08.12.2014 US 201462088815 P
(43) Date of publication of application: 18.10.2017
(73) Proprietor: Breas Medical, Inc., North Billerica, MA 01862 (US)
(72) Inventor: HARRISON, Donald, Charlestown, Massachusetts 02129 (US)
(74) Representative: Appleyard Lees IP LLP
(86) International application number: PCT/US2015/064514
(87) International publication number: WO 2016/094411

(56) References cited:
- WO-A1-2013/108145
- WO-A2-2007/115153
- US-A1- 2012 318 272

## Description

### PRIORITY CLAIM

Priority is claimed to co-pending U.S. Provisional Patent Application Serial No. 62/088,815, filed December 8th, 2014.

### COPYRIGHT STATEMENT

A portion of the disclosure of this patent application document contains material that is subject to copyright protection including the drawings. The copyright owner has no objection to the facsimile reproduction by anyone of the patent document or the patent disclosure as it appears in the Patent and Trademark Office file or records, but otherwise reserves all copyright rights whatsoever.

### BACKGROUND OF THE INVENTION

### 1. Field of the Invention

The present invention relates to medical devices, and, more particularly to facial or nasal masks and headgear portions of air delivery devices that assist with the delivery of gas to the breathing airways of users. These mask and headgear systems and devices can be used with positive airway pressure [PAP] such as continuous positive airway pressure devices [CPAP], automatic positive airway pressure devices [APAP], variable positive airway pressure devices [VPAP], and bi-level positive airway pressure devices [BPAP].

### 2. Description of the Prior Art

Previous masks have been bulky, heavy, and/or difficult to secure over a user's breathing airways. Additionally, the pressure on and around the face required to maintain a bulky positive air pressure mask system in place can be uncomfortable.

US 2012/318272 is background art and discloses a cushion inside a cushion patient interface. WO2007/115153 is background art and discloses an adjustable CPAP mask assembly. WO2013/108145 is background art and discloses a cushion with venting support for use with a patient interface device.

The present invention seeks to address these concerns by providing a lighter weight mask system that is secured around the mouth and nasal area with less pressure while maintaining a good seal for use with PAP devices.

### SUMMARY OF THE INVENTION

In one embodiment a positive airway interface system as set out in accompanying claim 1 is contemplated.

In some embodiments a plurality of attachment mechanisms can be provided about an external surface of the external shell and configures such that when a force applied to the attachment mechanisms the force is transformed in a uniform-like manner through the internal support structure to sealingly engage the user interface aperture around a user's mouth and nose. In some such embodiments the attachment mechanisms can be formed of a plurality of circular grommets being configured to affix to corresponding loops of a headgear assembly.

The mask can further include a user interface aperture for interfacing with the user's face and an inlet aperture for receiving a supply of pressurized air.

In some embodiments an input adapter can be provided which is configured to couple to the inlet aperture of the facial mask, the input adapter having a higher durometer than the facial mask.

In yet additional embodiments a removable heat moisture exchange (HME) can be provided and disposed within the input adapter or within the mask itself.

In yet other embodiments one or more CO₂ washout vents can be provided through the input adapter or the mask. Such CO₂ washout vent(s) can be provided in a removable insert which covers a front aperture of the input adapter.

In yet additional embodiments a rigid coupler can be provided about the inlet aperture of the facial mask so as to form a secure attachment means for the input adapter.

In yet additional embodiments an anti-asphyxia valve can be provided through the input adapter.

In some alternative embodiments the mask can include one or more malleable sealing membranes disposed about the user interface aperture of the facial mask which sealing membranes can include at least a first malleable sealing membrane extending inwardly into an internal cavity of the facial mask from an external edge of the internal support structure; and a second malleable sealing membrane extending inwardly into the internal cavity of the facial mask from an internal edge of the internal support structure.

In some embodiments the mask can further include a support membrane extending from a first point of an outer circumference of the facial mask to a second point of the outer circumference of the facial mask, the support membrane configured to rest on a maxilla portion of the user's face between the user's nose and mouth.

In yet alternative embodiments, a method of using a semi-flexible airway interface system is contemplated which includes the steps of: placing a facial mask system over a user's nose and mouth; affixing the facial mask system about the user's nose and mouth using a strap that connects to the attachment mechanisms and extends around the user's head; wherein a force applied to the attachment mechanisms via the strap causes the interface aperture to sealingly engage around the user's breathing airways.

These and other embodiments are described in more detail herein.

### BRIEF DESCRIPTION OF THE DRAWINGS

The foregoing and other objects, aspects, features, and advantages of the disclosure will become more apparent and better understood by referring to the following description taken in conjunction with the accompanying drawings, in which:
Fig. 1 illustrates a perspective view of a positive airway interface system being worn by a user;

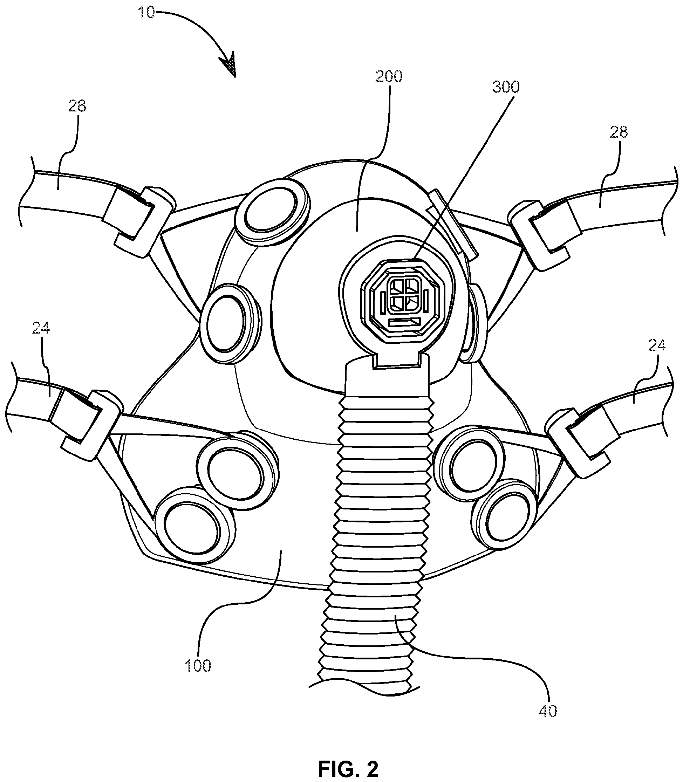

Fig. 2 illustrates an alternative perspective view of the positive airway interface system of FIG. 1;
as well as a perspective view without the user;
Figs. 3A-B illustrate front and rear perspective views of a semi-flexible facial mask for use in the positive airway interface system of FIG. 1;
Fig. 3C illustrates a rear perspective view of an alternate semi-flexible facial mask where the user interface is divided into two apertures - a nasal aperture and oral aperture;
FIGs. 4A-B illustrate side cross sectional views illustrating various sealing membranes and internal support structures for use with the semi-flexible facial masks of FIG. 3;
FIG. 5 illustrates a perspective view of a rigid coupler for coupling the semi-flexible facial mask to an input adapter;
FIGs 6A-B illustrate an exploded perspective view and a bottom view of an input adapter for use with the positive airway interface system of FIG. 1; and
FIG. 7 illustrates a perspective view of a removable heat moisture exchange adaptable for use in any of the embodiments shown.

### DETAILED DESCRIPTION OF THE PREFERRED EMBODIMENT

To provide an overall understanding of the systems, devices, and methods described herein, certain illustrative embodiments will be described. Although the embodiments and features described herein are frequently described for use in connection with CPAP apparatuses, systems, and methods, it will be understood that all the components, mechanisms, systems, methods, and other features outlined below can be combined with one another in any suitable manner and can be adapted and applied to other PAP apparatuses, systems, and methods, including, but not limited to, APAP, VPAP, and BPAP apparatuses, systems, and methods.

The present application seeks to provide a solution to the aforementioned problems by creating an adjustable and comfortable mask assembly system that has interchangeable components, is light-weight, and provides a uniform sealing around an area surrounding a user's nose and mouth that is enabled by a unique internal support structure.

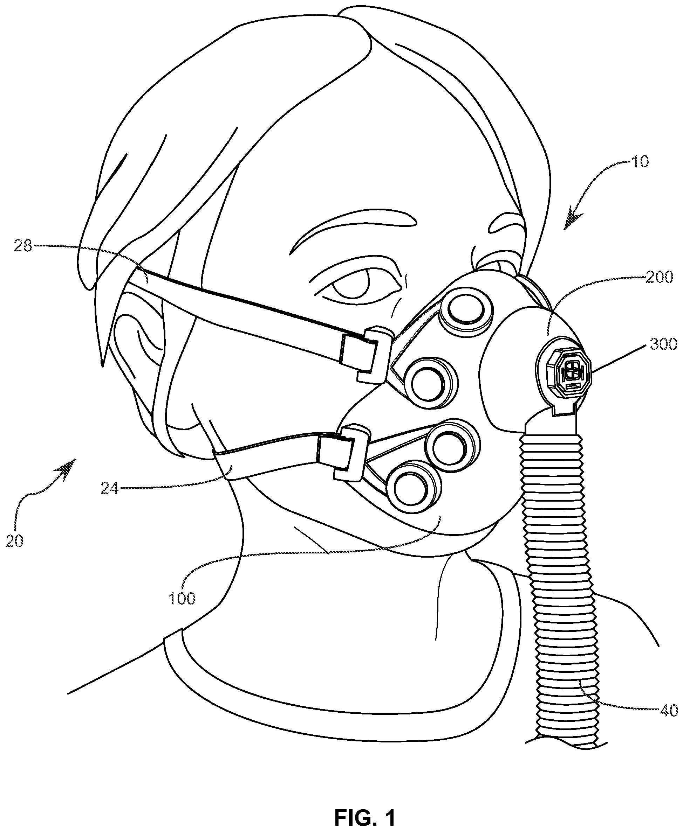

Figs. 1A-B illustrate positive airway interface system 10, which can include a headgear or strap assembly 20 which is configured to connect to a facial mask 100. The mask 100 can be configured, as shown, to interface with a user's face over both the user's nose and mouth so as to sealingly engage over the user's air passageways. The mask can then be provided with a positive air pressure adapter 200 which receives a pressurized flow of air through an air supply tube 40 and delivers it to the mask 100.

In some embodiments the strap assembly 20 can include a lower head strap 24 configured to apply a lower sealing force between a lower portion of the mask 100 and around the user's mouth, additionally the strap assembly 20 can include an upper head strap 28 which is configured to apply an upper sealing force between an upper portion of the mask 100 and around the user's nose. It will be appreciated that the air supply tube 40 can be attached to a blower, or other positive air pressure supply as necessary. In this manner positive air pressure can be supplied into the mask assembly, and thereby into the airways of the user.

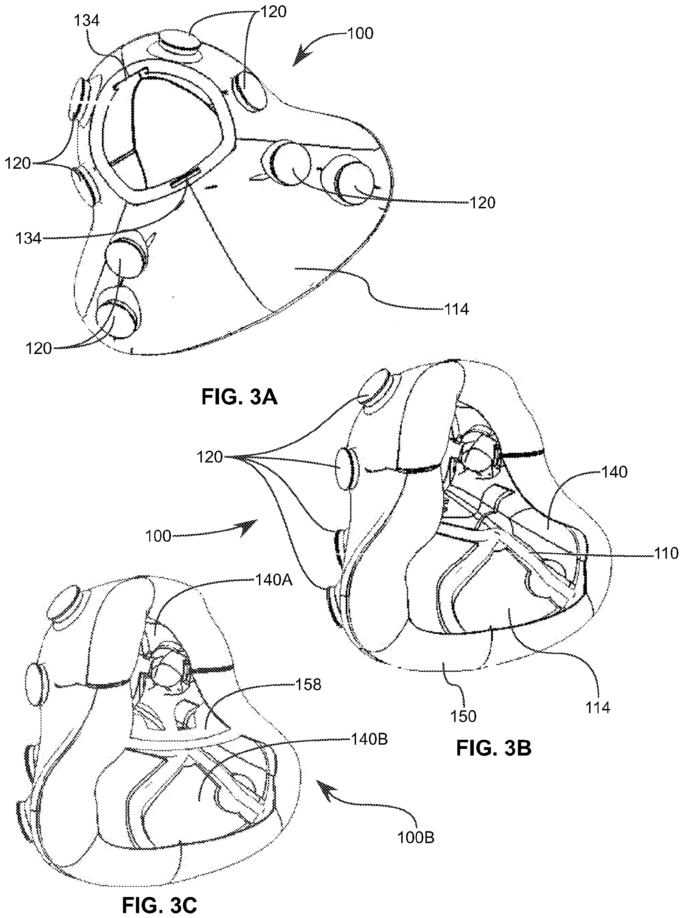

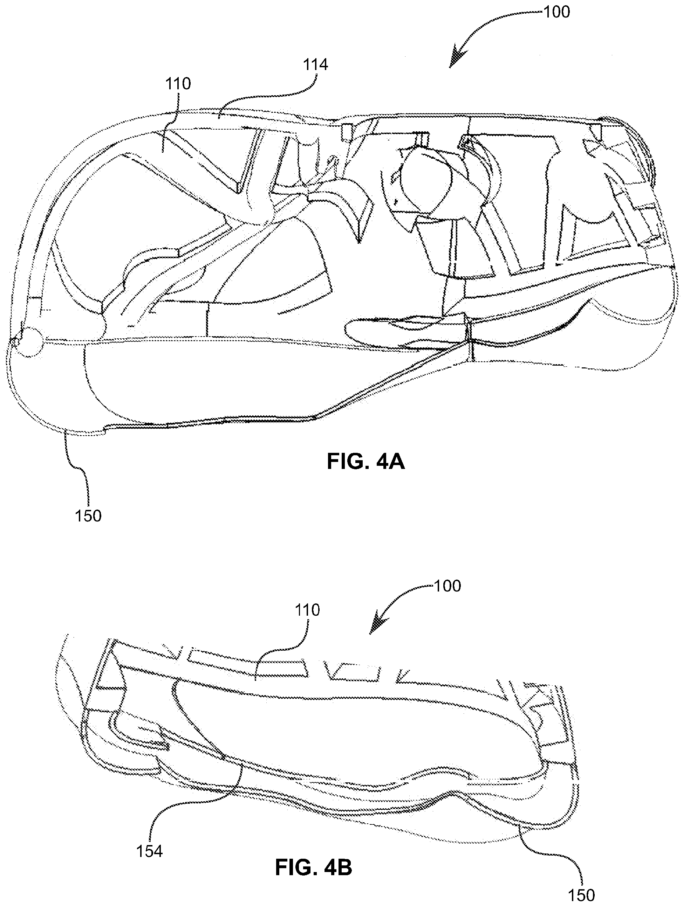

As shown in FIGs. 3A-C and FIGs.4A-B, the facial mask 100 can include an internal support structure 110 which is then sealed by providing an external shell 114 which can be disposed over, unitarily with, or overmolded upon, the internal support structure 110 so as to form a sealed internal cavity. The user's mouth and nose can then be placed within the internal cavity so as to receive the supply of pressurized gas into the user's air passageways.

A plurality of attachment mechanisms 120 can be provided about an external surface of the external shell 114 which extend through the external shell and connecting with the internal support structure 110. The attachment means 120 can be provided as raised or angled plateaus having mushroomed or flaring tops. Attachment means of this style allow for a snap or interference fit between each of the plateaus and loops provided on the headgear straps 20, as shown in FIGs. 1. In this manner the loops can snap over and interferingly engage each of the raised plateaus, such that the loops resist popping off or otherwise disengaging from the attachment means 120.

A user interface aperture 140 can be provided to the mask 100 which is configured to provide a seal around the user's mouth and nose by transmitting a tensile sealing force applied the strap assembly 20, as shown in FIG. 1, through the attachment mechanisms 120, into the internal support structure 110 and finally onto a sealing membrane 150. The sealing membrane 150 can extend from the outer shell 114 or the internal support structure from an outer perimeter of the user interface aperture 140 toward a central portion of the user interface aperture. The sealing membrane can be formed of a thin and malleable substance which can deform so as to conform to the contours of the user's face.

It will be appreciated that the mask 100 can be formed such that the mask is semi flexible, so as to provide a comfortable fit about the user's face, wherein the internal support structure provides sufficient rigidity so as to not collapse under the sealing force applied by the strap assembly, but is not so rigid so as to cause uncomfortable pressure points about the user's face.

The internal support structure 110 operates to suspend and provide support to the relatively flexible and thin outer shell 114 which would otherwise have insufficient structural support. This combination between the internal support structure 110 and the thin outer shell 114 allows for a thinner outer shell 114 to be suspended between the interconnected truss or web structure of the internal support structure 110 and thus allows for a thinner exterior wall and ultimately a lighter and thus more comfortable mask overall.

It will then be further appreciated that because the mask 100 is semi-flexible it can thus be difficult to couple with because this flexibility can cause anything attached thereto to be easy to pull away from the mask, i.e. an otherwise relatively rigid input adapter 200. For this reason, a rigid coupler 250 can be provided between the input adapter 200 and the mask 100 so as to facilitate a more secure connection between the two. In the embodiments shown, the rigid coupler 250 is intended to serve as a more secure attachment means between the inlet adapter 200 and the semi-flexible mask 100. As such, the rigid coupler 250 can be permanently, unitarily formed with, or otherwise provided with a secure connection to the semi-flexible mask 100. In some embodiments the rigid coupler 250 can be provided with a sealing channel 254 which can interface with a corresponding protrusion on either the mask or the input adapter so as to provide an air tight seal therewith.

The rigid coupler 250 can further be provided with clip slots 258 for interfacing with corresponding clips of the inlet adapter 200, or alternatively the clip slots 258 can merely be pass through slots allowing the clip protrusions of the inlet adapter to interface directly with the corresponding clip interfaces 134 of the mask 100 itself.

Additionally, the mask 100 can be provided with a flat or otherwise consistent bonding surface for bonding to the rigid coupler 250 using adhesives or other suitable bonding agents. Alternatively, the mask 100 can be overmolded over the coupler 250 so as to integrate the coupler 250 into the inlet aperture 130. If the rigid coupler 250 is formed unitarily or overmolded by the mask 100 it will then be unnecessary for both the mask and the rigid coupler to have corresponding clip interfaces.

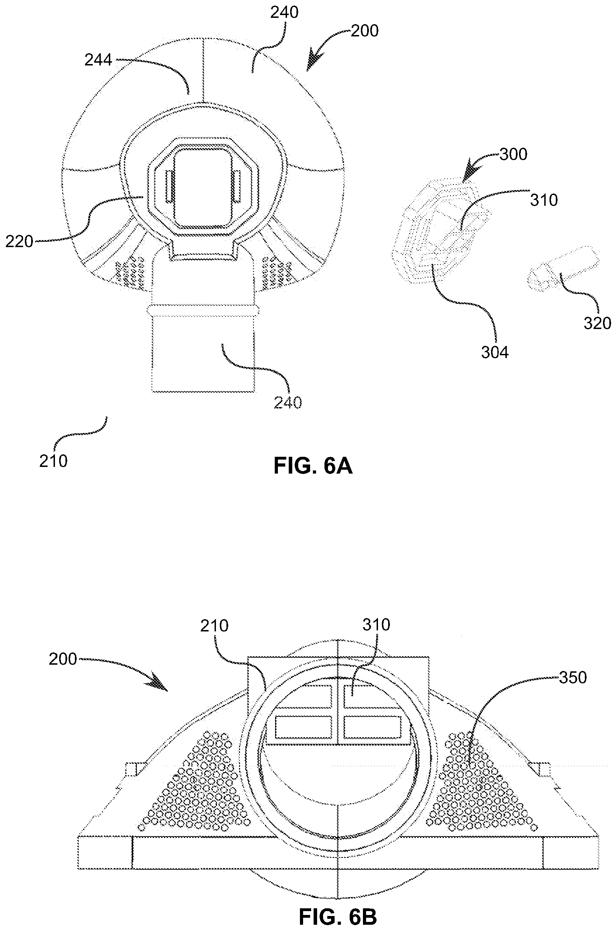

Figures 6A-B illustrate an exploded rear isometric view and a bottom view respectively of an exemplary input adapter 200 being configured to couple to the inlet aperture of the mask 100, the input adapter 200 having a higher durometer than the mask 100. The inlet adapter 200 can include a hose adapter 210 which can be configured to attach to a positive pressure air supply (not shown) so as to facilitate the provision of a pressurized air flow into the internal cavity of the mask. The inlet adapter 200 can also include a plurality of CO₂ washout vents 350 which can be configured in various orientations and number so as to achieve a suitable dissipation of CO₂ exhaled by the user into the mask assembly. It will be appreciated that these CO₂ washout vents 350 are shown herein in the bottom portion of the adapter, but can also be provided in the front or top surfaces as well. Alternatively, these washout vents can also be provided through the outer shell 114 of the mask 100 so as to achieve a proper amount of CO₂ venting.

The CO₂ exhaust ports can be provided with interchangeable/replaceable CO₂ venting material. By choosing different materials having different permeability the user can thus choose the degree of venting that is preferable. In some instances, the material is knife-coated silicone material and the amount of knife-coating alters the permeability of the material. In some embodiments the fixed CO₂ exhaust is not removable and is permanent to the front adaptor. In these fixed embodiments, the CO₂ venting material can be over molded with the front adaptor. While the hexagonal shape shown in the depicted embodiment, it will be appreciated that circular, square, or any other shapes can be used.

The inlet adapter 200 can also be provided with one or more attachment clips 240 for facilitating the coupling of the inlet adapter 200 to either the mask 100 or the rigid coupler 250. It will be appreciated that while these attachment clips 240 are shown as a male component of an interference latch system, any number of attachment means and orientations will be recognized by those having skill in the art as being suitable for providing a compressive sealing force between the inlet adapter and an inlet aperture of the mask.

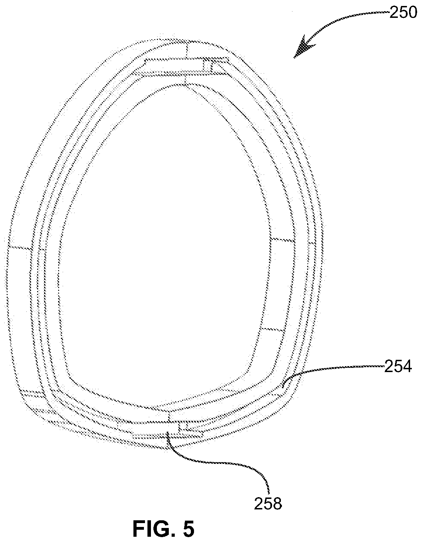

Additionally, in the embodiment shown, the inlet adapter 200 can be provided with a sealing lip 244 which will interface with a sealing channel 254 of the coupler 254, as seen in FIG. 5. This sealing channel 254 and sealing lip 244 correspond in shape so as to provide an air tight seal between the inlet adapter 200 and the coupler 250.

The inlet adapter 200 can further be provided with a venting aperture 220 provided therein. The venting aperture 220 can be provided with an anti-asphyxia valve 300 having an insert body 304 and an aperture 310 provided therethrough. A valve flap 320 can be attached to the insert body 304 and be configured to selectively close the aperture 310 in response to air flow through the inlet adapter 200. For example, flowing air coming from the tube adapter will push the flap upwards, thus closing the aperture 210, however, when air is not flowing, the flap 320 can fall downward so as to open the aperture 210 and thus allow the user to breath fresh non-pressurized air from outside the mask through the aperture.

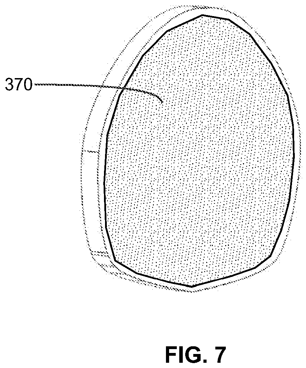

In some embodiments, and as shown in FIG. 7 a heat moisture exchange (HME) can be provided in the form of a porous member 370. The HME 370 can be formed of varying materials, porous, wicking, or otherwise so as to retain exhaled moisture within the mask 100 or within the input adapter 200. This HME 370 can be shaped so as to correspond in shape to the interior of the mask or input adapter and cover or force the air supply to pass therethrough such that the trapped moisture can then be partially evaporated or entrained into the inlet air supply and thus increase the moisture content of the air supply and reduce the harshness of the supplied air. This HME 370 can be formed in varying shapes and thicknesses so as to be adaptable for use in either of the input adapters disclosed herein, or alternatively, within the nasal mask cavity.

In some embodiments the attachment mechanisms can be formed as a plurality of circular grommets being configured to affix to corresponding loops of a headgear assembly. In this manner the connection between the circular grommets and the corresponding loops can have a rotational degree of freedom so as to increase the potential adjustability of the fit.

In yet additional embodiments the mask can be provided with a plurality of malleable sealing membranes 150 and 154 being disposed about the user interface aperture of the nasal mask. The first malleable sealing membrane 150 can be provided so as to extend inwardly into an internal cavity of the mask from an external edge of the internal support structure 110 and the second malleable sealing membrane 154 can be provided so as to extend inwardly into the internal cavity of the mask from an internal edge of the internal support structure 110.

It will be appreciated that individual users will often have different sized faces, thus the mask 100 as shown herein can be scaled to fit various users wherein the rigid coupler 250 remains constant in size so as to facilitate a universal fit with a common input adapter 200.

Further, a central support membrane 158, as shown in an alternative mask 100B in FIG. 3C, can be provided such that a sealing membrane flap can be provided which seals both ways, i.e. towards mouth as well as towards nasal portion. In effect, the central support membrane 158 divides the user interface into two portions 140a and 140b, where 140a engages around a user's nasal region and 140b engages around a user's mouth or oral region. It will be appreciated that the center support membrane 158 can also be removable in an alternative embodiment wherein both the mouth and nose of the user extend into a common internal cavity. The central support membrane 158 can serve to maintain a certain degree of structural integrity to the interior of the mask 100B by limiting the circumferential expansion of the mask when pressurized air is being supplied thereto. The central support membrane 158 can rest on the maxilla of the patient between the upper lip and the nose, which secants or divides the two circumferential sides of the mask and can thus maintain the sealing membrane 150 in an interior direction even under pressure.

The above embodiments can be formed of various materials include silicone materials, plastics, and the like. Furthermore, the durometer of each of the materials can be varied. For example, the durometer of the outer shell can be more pliable compared to the internal support structure. Alternatively, the thicknesses of the internal support structure can vary to provide the necessary mechanical coupling of a force being applied to the attachment means.

The above description is merely illustrative. Having thus described several aspects of at least one embodiment of this invention including the preferred embodiments, it is to be appreciated that various alterations, modifications, and improvements will readily occur to those skilled in the art. Such alterations, modifications, and improvements are intended to be part of this disclosure, and are intended to be within the scope of the invention. Accordingly, the foregoing description and drawing are by way of example only.

## Claims

1. A positive airway interface system (10) comprising:
a facial mask (100), the facial mask comprising:
an internal support structure (110);
an external shell (114) disposed over the internal support structure (110);
a plurality of strap attachment means (120) provided about an external surface of the external shell (114);
a user interface aperture (140); and
an inlet aperture;
**characterised in that** each of the plurality of strap attachment means (120) extends through the external shell (114) so as to mechanically couple to the internal support structure (110).

2. The positive airway interface system of claim 1, comprising a
n input adapter (200) being configured to couple to the inlet aperture of the facial mask, the input adapter (200) having a higher durometer than the facial mask.

3. The positive airway interface system of claim 2, comprising a removable heat moisture exchange (370) disposed within the input adapter.

4. The positive airway interface system of claim 2, comprising one or more CO₂ washout vents (350) being provided through the input adapter.

5. The positive airway interface system of claim 4, wherein the CO₂ washout vent(s) are provided in a removable insert which covers a front aperture of the input adapter.

6. The positive airway interface system of claim 2, comprising a rigid coupler (250) provided about the inlet aperture of the facial mask.

7. The positive airway interface system of claim 2, comprising an anti-asphyxia valve (300) provided through the input adapter.

8. The positive airway interface system of claim 1, wherein the strap attachment means (120) (120) are formed of a plurality of circular grommets being configured to affix to a corresponding plurality of loops provided about a headgear assembly.

9. The positive airway interface system of claim 1, comprising one or more malleable sealing membranes (150, 154) disposed about the user interface aperture of the facial mask.

10. The positive airway interface system of claim 9, comprising:
a first malleable sealing membrane (150) extending inwardly into an internal cavity of the facial mask from an external edge of the internal support structure; and
a second malleable sealing membrane (154) extending inwardly into the internal cavity of the facial mask from an internal edge of the internal support structure.

11. The positive airway interface system of claim 9, comprising a support membrane (158) extending from a first point of an outer circumference of the facial mask to a second point of the outer circumference of the facial mask, the support membrane configured to rest on a maxilla portion of the user's face between the user's nose and mouth.

12. The positive airway interface system of claim 1, wherein the internal support structure (110) is formed of interconnected truss or web features.

13. A facial mask comprising:
an internal support structure (110);
an external shell (114) disposed over the internal support structure (110);
a plurality of strap attachment means (120) provided about an external surface of the external shell (114);
a user interface aperture (140); and an inlet aperture;
**characterized in that** each of the plurality of strap attachment means (120) extends through the external shell (114) so as to mechanically couple to the internal support structure (110).

14. The facial mask of claim 13, comprising one or more features, each independently selected from
(i) a removable HME unit disposed towards the front portion of the mask; or
(ii) one or more CO₂ vents; or
(iii) one or more straps or headgear systems that secure to the attachment means; or
(iv) one or more sealing membranes (150 154).

15. The facial mask of claim 14, wherein a first of the one or more sealing
membranes (150, 154) comprise malleable sealing membranes, and wherein a first malleable sealing membrane (150, 154) extends from an external edge of the internal support structure inwardly into an internal cavity of the mask, and a second malleable sealing membrane (150, 154) extends from an internal edge of the internal support structure inwardly into an internal cavity of the mask.

## Patentansprüche

1. Positives Luftwegschnittstellensystem (10), umfassend:
eine Gesichtsmaske (100), wobei die Gesichtsmaske Folgendes umfasst:
eine interne Stützstruktur (110);
eine Außenhülle (114), die über der internen Stützstruktur (110) angeordnet ist;
eine Vielzahl von Bandbefestigungsmitteln (120), die um eine Außenfläche der Außenhülle (114) angeordnet ist;
eine Benutzerschnittstellenöffnung (140); und
eine Einlassöffnung;
**dadurch gekennzeichnet, dass** sich jedes von der Vielzahl von Bandbefestigungsmitteln (120) durch die Außenhülle (114) erstreckt, um mechanisch an die interne Stützstruktur (110) gekoppelt zu werden.

2. Positives Luftwegschnittstellensystem nach Anspruch 1, umfassend einen Eingangsadapter (200), der dazu konfiguriert ist, an die Einlassöffnung der Gesichtsmaske gekoppelt zu werden, wobei der Eingangsadapter (200) einen höheren Durometerwert aufweist als die Gesichtsmaske.

3. Positives Luftwegschnittstellensystem nach Anspruch 2, umfassend einen entfernbaren Wärme-Feuchte-Tauscher (370), der innerhalb des Eingangsadapters angeordnet ist.

4. Positives Luftwegschnittstellensystem nach Anspruch 2, umfassend eine oder mehrere CO₂-Auswaschentlüftungen (350), die durch den Eingangsadapter bereitgestellt sind.

5. Positives Luftwegschnittstellensystem nach Anspruch 4, wobei die CO₂-Auswaschentlüftung(en) in einem entfernbaren Einsatz bereitgestellt ist/sind, der eine vordere Öffnung des Eingangsadapters abdeckt.

6. Positives Luftwegschnittstellensystem nach Anspruch 2, umfassend ein starres Verbindungselement (250), das um die Einlassöffnung der Gesichtsmaske bereitgestellt ist.

7. Positives Luftwegschnittstellensystem nach Anspruch 2, umfassend ein Anti-Asphyxie-Ventil (300), das durch den Eingangsadapter bereitgestellt ist.

8. Positives Luftwegschnittstellensystem nach Anspruch 1, wobei die Bandbefestigungsmittel (120) aus einer Vielzahl von kreisförmigen Tüllen gebildet sind, die dazu konfiguriert ist, an einer entsprechenden Vielzahl von Schleifen befestigt zu werden, die um eine Kopfgestellbaugruppe bereitgestellt ist.

9. Positives Luftwegschnittstellensystem nach Anspruch 1, umfassend eine oder mehrere verformbare Dichtungsmembranen (150, 154), die um die Benutzerschnittstellenöffnung der Gesichtsmaske angeordnet ist/sind.

10. Positives Luftwegschnittstellensystem nach Anspruch 9, umfassend:
eine erste verformbare Dichtungsmembran (150), die sich nach innen in einen internen Hohlraum der Gesichtsmaske von einer Außenkante der internen Stützstruktur erstreckt; und
eine zweite verformbare Dichtungsmembran (154), die sich nach innen in den internen Hohlraum der Gesichtsmaske von einer Innenkante der internen Stützstruktur erstreckt.

11. Positives Luftwegschnittstellensystem nach Anspruch 9, umfassend eine Stützmembran (158), die sich von einem ersten Punkt eines Außenumfangs der Gesichtsmaske zu einem zweiten Punkt des Außenumfangs der Gesichtsmaske erstreckt, wobei die Stützmembran dazu konfiguriert ist, auf einem Oberkieferabschnitt des Gesichts des Benutzers zwischen der Nase und dem Mund des Benutzers zu ruhen.

12. Positives Luftwegschnittstellensystem nach Anspruch 1, wobei die interne Stützstruktur (110) aus miteinander verbundenen Träger- oder Stegmerkmalen gebildet ist.

13. Gesichtsmaske, umfassend:
eine interne Stützstruktur (110);
eine Außenhülle (114), die über der internen Stützstruktur (110) angeordnet ist;
eine Vielzahl von Bandbefestigungsmitteln (120), die um eine Außenfläche der Außenhülle (114) bereitgestellt ist;
eine Benutzerschnittstellenöffnung (140); und
eine Einlassöffnung;
**dadurch gekennzeichnet, dass** sich jedes von der Vielzahl von Bandbefestigungsmitteln (120) durch die Außenhülle (114) erstreckt, um mechanisch an die interne Stützstruktur (110) gekoppelt zu werden.

14. Gesichtsmaske nach Anspruch 13, umfassend ein oder mehrere Merkmale, von denen jedes unabhängig ausgewählt ist aus
(i) einer entfernbaren HME-Einheit, die in Richtung des Vorderabschnitts der Maske angeordnet ist; oder
(ii) einer oder mehreren CO₂-Entlüftungen; oder
(iii) einem oder mehreren Bändern oder Kopfgestellsystemen, das/die an den Befestigungsmitteln gesichert ist/sind; oder
(iv) einer oder mehreren Dichtungsmembranen (150, 154).

15. Gesichtsmaske nach Anspruch 14, wobei eine erste der einen oder der mehreren Dichtungsmembranen (150, 154) verformbare Dichtungsmembranen umfasst, und wobei sich eine erste verformbare Dichtungsmembran (150, 154) von einer Außenkante der internen Stützstruktur nach innen in einen internen Hohlraum der Maske erstreckt und sich eine zweite verformbare Dichtungsmembran (150, 154) von einer Innenkante der internen Stützstruktur nach innen in einen internen Hohlraum der Maske erstreckt.

## Revendications

1. Système d'interface positive des voies respiratoires (10) comprenant :
un masque facial (100), le masque facial comprenant :
une structure de support interne (110) ;
une coque externe (114) disposée sur la structure de support interne (110) ;
une pluralité de moyens de fixation de sangle (120) disposés autour d'une surface externe de la coque externe (114) ;
une ouverture d'interface utilisateur (140) ; et
une ouverture d'admission ;
**caractérisé en ce que** chacun de la pluralité de moyens de fixation de sangle (120) s'étend à travers la coque externe (114) de façon à s'accoupler mécaniquement à la structure de support interne (110).

2. Système d'interface positive des voies respiratoires selon la revendication 1, comprenant un adaptateur d'entrée (200) configuré pour s'accoupler à l'ouverture d'admission du masque facial, l'adaptateur d'entrée (200) présentant une dureté plus élevée que le masque facial.

3. Système d'interface positive des voies respiratoires selon la revendication 2, comprenant un dispositif d'échange thermohydrique (370) amovible disposé dans l'adaptateur d'entrée.

4. Système d'interface positive des voies respiratoires selon la revendication 2, comprenant un ou plusieurs évents d'évacuation de CO₂ (350) disposés à travers l'adaptateur d'entrée.

5. Système d'interface positive des voies respiratoires selon la revendication 4, dans lequel le ou les évents d'évacuation de CO₂ sont disposés dans un insert amovible qui couvre une ouverture avant de l'adaptateur d'entrée.

6. Système d'interface positive des voies respiratoires selon la revendication 2, comprenant un coupleur rigide (250) disposé autour de l'ouverture d'admission du masque facial.

7. Système d'interface positive des voies respiratoires selon la revendication 2, comprenant une valve anti-asphyxie (300) disposée à travers l'adaptateur d'entrée.

8. Système d'interface positive des voies respiratoires selon la revendication 1, dans lequel les moyens de fixation de sangle (120) sont formés d'une pluralité d'œillets circulaires configurés pour se fixer à une pluralité correspondante de boucles disposées autour d'un ensemble harnais.

9. Système d'interface positive des voies respiratoires selon la revendication 1, comprenant une ou plusieurs membranes d'étanchéité malléables (150, 154) disposées autour de l'ouverture de l'interface utilisateur du masque facial.

10. Système d'interface positive des voies respiratoires selon la revendication 9, comprenant :
une première membrane d'étanchéité malléable (150) s'étendant vers l'intérieur dans une cavité interne du masque facial depuis un bord externe de la structure de support interne ; et
une deuxième membrane d'étanchéité malléable (154) s'étendant vers l'intérieur dans une cavité interne du masque facial depuis un bord interne de la structure de support interne.

11. Système d'interface positive des voies respiratoires selon la revendication 9, comprenant une membrane de support (158) s'étendant depuis un premier point d'une circonférence extérieure du masque facial vers un deuxième point de la circonférence extérieure du masque facial, la membrane de support étant configurée pour reposer sur une partie maxillaire du visage de l'utilisateur entre le nez et la bouche de l'utilisateur.

12. Système d'interface positive des voies respiratoires selon la revendication 1, dans lequel la structure de support interne (110) est formée d'éléments de bande ou d'armature interconnectés.

13. Masque facial comprenant :
une structure de support interne (110) ;
une coque externe (114) disposée sur la structure de support interne (110) ;
une pluralité de moyens de fixation de sangle (120) disposés autour d'une surface externe de la coque externe (114) ;
une ouverture d'interface utilisateur (140) ; et
une ouverture d'admission ;
**caractérisé en ce que** chacun de la pluralité de moyens de fixation de sangle (120) s'étend à travers la coque externe (114) de façon à s'accoupler mécaniquement à la structure de support interne (110).

14. Masque facial selon la revendication 13, comprenant un ou plusieurs éléments, chacun indépendamment sélectionné parmi
(i) une unité HME amovible disposée vers la partie avant du masque ; ou
(ii) un ou plusieurs évents à CO₂ ; ou
(iii) un ou plusieurs systèmes de harnais ou de sangles qui se fixent sur les moyens de fixation ; ou
(iv) une ou plusieurs membranes d'étanchéité (150, 154).

15. Masque facial selon la revendication 14, dans lequel une première de la ou des membranes d'étanchéité (150, 154) comprend des membranes d'étanchéité malléables, et dans lequel une première membrane d'étanchéité malléable (150, 154) s'étend depuis un bord externe de la structure de support interne vers l'intérieur dans une cavité interne du masque, et une deuxième membrane d'étanchéité malléable (150, 154) s'étend depuis un bord interne de la structure de support interne vers l'intérieur dans une cavité interne du masque.
